# DEMANDE DE BREVET EUROPEEN

(11) **EP 1 862 161 A1**
(43) Date de publication de la demande: **05.12.2007**
(21) Numéro de dépôt: 07010934.3
(22) Date de dépôt: 04.06.2007
(51) Int. Cl.: A61K 8/73, A61K 8/81, A61K 31/728, A61K 47/10, A61K 9/00

(54) **Compositions pharmaceutiques, cosmétiques et/ou dispositifs médicaux injectables à base d'acide hyaluronique**

(30) Priorité: 02.06.2006 FR 0604906
(71) Demandeur: Labotaroire Genevrier, 06901 Sophia Antipolis Cedex (FR)
(72) Inventeur: Vacher, Dominique, 06250 Mougins (FR)
(74) Mandataire: Burtin, Jean-François

(57) **Abrégé**

La présente invention se rapporte au domaine de la thérapeutique, de la chirurgie plastique et de la cosmétique.

L'invention concerne en particulier des préparations injectables viscoélastiques contenant de l'acide hyaluronique ou un de ses sels et un copolymère en blocs polyoxyéthylène-polyoxypropylène commercialisé sous la dénomination Poloxamer®) 407 (Lutrol F 117 BASF) en quantité appropriée pour former une solution dont la viscosité va en décroissant avec l'abaissement de la température, de telle sorte que leur injection dans une articulation ou sous la peau se fasse plus facilement.

Utilisation de ces compositions en injections intra-articulaires ou sous-cutanées, à des fins thérapeutiques ou cosmétiques.

## Description

La présente invention se rapporte au domaine de la thérapeutique et plus particulièrement au domaine du médicament, et du dispositif médical notamment pour le traitement des troubles articulaires.

La présente invention se rapporte également aux domaines de la chirurgie plastique et de la cosmétique, en particulier pour traiter ou compenser les ridules, les rides, les cicatrices et faire disparaître les séquelles d'interventions chirurgicales.

L'invention concerne des compositions injectables à base d'acide hyaluronique. En effet, les injections intra-articulaires ou subdermiques de formulations pharmaceutiques et les dispositifs médicaux à base d'acide hyaluronique connaissent actuellement un net accroissement de leurs utilisations dans le domaine des troubles articulaires, notamment des manifestations douloureuses ou invalidantes de la gonarthrose. On utilise l'acide hyaluronique pour des injections ou des infiltrations dans la capsule du genou ou dans le liquide synovial pour compenser des pertes de liquide dues à la diffusion intra capsulaire du liquide synovial ou à un mauvais état de la capsule.

Des brevets récents font également état de l'utilisation de compositions injectables contenant de l'acide hyaluronique pour le traitement des traumatismes du genou avec synovite et pour la régénération du cartilage (EP 1.560.588) ainsi que pour le traitement de l'arthrite rhumatoïde (EP 1.166.788).

L'acide hyaluronique est un polysaccharide endogène, de haute masse moléculaire (50.000 Da à 200.000 Da) formé d'unités répétitives d'acide glucuronique et de N-acétyl glucosamine. Il joue un rôle de premier plan en tant que constituant du liquide synovial en raison des propriétés viscoélastiques, lubrifiantes et anti-inflammatoires de ce dernier. C'est le liquide synovial qui assure la motricité de l'articulation du genou dont dépend l'aptitude à la marche. L'acide hyaluronique intervient également dans le maintien de la teneur en eau et de la viscosité de la matrice cartilagineuse.

Les injections intra-articulaires d'acide hyaluronique (visco-supplémentation et visco-induction) sont efficaces dans les cas de pertes de liquide synovial par diffusion et/ou endommagement de la capsule du genou. L'acide hyaluronique intervient également dans la réparation du cartilage souvent endommagé ou lésé à la suite d'un traumatisme, comme un accident articulaire lié au sport. L'injection d'acide hyaluronique, en raison de sa forte viscoélasticité, apporte une sensation de soulagement immédiat lors du fonctionnement de l'articulation, notamment celle du genou. L'acide hyaluronique a également été décrit comme exerçant une stimulation des synthèses au niveau des synoviocytes et des chondrocytes, qui permet à moyen terme d'envisager une préservation de l'état du cartilage articulaire.

Lors de son utilisation en injection intra-articulaire, l'acide hyaluronique diffuse rapidement dans les couches superficielles du cartilage. Sa demi-vie est de 6 à 8 heures seulement dans le liquide synovial. De ce fait il est recommandé de pratiquer de 3 à 5 injections à une semaine d'intervalle pour s'assurer de sa rémanence dans l'articulation et donc de l'efficacité du traitement.

Cette rapide disparition de l'acide hyaluronique dans la capsule articulaire résulte soit des fuites de liquide par compression, soit de la dépolymérisation de l'acide hyaluronique, modifiant sa viscosité. C'est pourquoi il est apparu souhaitable de complémenter les compositions injectables d'acide hyaluronique sans en modifier les effets viscoélastiques.

On pouvait en particulier penser additionner l'acide hyaluronique de composés de forte viscosité pour maintenir *in situ* un effet de visco-supplémentation et empêcher la dégradation de l'acide hyaluronique en dehors du cartilage. Le composé à forte viscosité jouerait alors un rôle de prothèse liquide qui renforcerait les propriétés de l'acide hyaluronique. Le nombre d'injections d'acide hyaluronique dans les différentes capsules articulaires s'en trouverait diminué, ce qui serait grandement avantageux.

Néanmoins l'augmentation de la viscosité d'une telle préparation nécessite l'utilisation d'une aiguille de section plus importante ce qui rend l'injection difficile pour l'opérateur et douloureuse pour le patient.

L'objet de l'invention est donc de réaliser une préparation injectable d'acide hyaluronique dont la rémanence soit plus grande et dont l'injection soit plus facile et peu douloureuse.

Ce problème a été résolu selon l'invention en additionnant l'acide hyaluronique d'un agent épaississant ou viscosifiant et filmogène. Cet agent épaississant est un copolymère en blocs polyoxyéthylène-polyoxypropylène de formule générale : 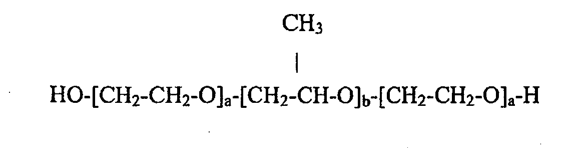 telle que a est environ 101 et b est environ 56.

La dénomination commune de ce composé est Poloxamer® 407.

Il s'agit d'un polymère dont la masse molaire s'échelonne de 9840 à 14600 g/mol. Il est soluble dans l'eau, l'éthanol et l'isopropanol.
Il est insoluble dans les solvants organiques comme l'éther, la paraffine et les huiles.

En solution dans l'eau, le Poloxamer® 407 se gélifie et forme une masse homogène et élastique. Ces gels sont thermoréversibles et manifestent un maximum de viscosité dans la gamme de 30 à 70° C. La température de transition dépend de la concentration en Poloxamer® 407. C'est en fonction de leur concentration et vers 50°C que les gels de Poloxamer® 407 perdent de leur viscosité. Au contraire, en dessous de 20°C-25°C la viscosité diminue considérablement. Un minimum de viscosité est atteint entre 0 et 10 °C.

En raison de ses propriétés tensioactives le Poloxamer® 407 a déjà été utilisé en association avec l'acide hyaluronique pour le nettoyage et l'entretien des lentilles de cornée (WO 00/60038). En outre pour diminuer la viscosité d'une telle préparation l'association Acide Hyaluronique (sous forme de sel de sodium) et de Poloxamer® 407 est additionné de Chlorure de sodium et de phosphate de sodium dibasique. Ainsi on obtient un effet de sel qui entraîne une diminution de la viscosité.

On a décrit également une préparation ophtalmique qui, instillée dans l'oeil à l'état liquide, se gélifie ensuite sur la cornée permettant ainsi une libération prolongé d'un principe actif (CN 1.377.706). Un autre brevet concerne une forme pharmaceutique à libération prolongée destinée à l'injection dans une tumeur d'un principe actif anticancéreux associé au Poloxamer® 407 (CN 1.385.154).

Le Poloxamer® 407 a déjà été associé à l'acide hyaluronique dans des microparticules destinées à franchir la barrière hépatique et à libérer ainsi un principe actif (DE 3700 911).

L'association d'acide hyaluronique et de Poloxamer® 407 pour des préparations injectables intra-articulaires n'avait jusqu'ici jamais été décrite.

L'objet de l'invention consiste donc en des compositions pharmaceutiques et/ou cosmétiques ou en des dispositifs médicaux, destinés à être injectés facilement dans les articulations ou sous la peau, caractérisés en ce qu'ils contiennent de l'acide hyaluronique ou l'un de ses sels et du Poloxamer® 407 dans un solvant approprié pour une injection intra-articulaire ou subdermique.

D'une manière préférentielle, le solvant utilisé est un solvant aqueux.

On a constaté que le refroidissement d'une telle association en dessous de 20° C ou de 25°C, en particulier en dessous de 10°C - 20°C, entraînait une baisse sensible de la viscosité. Il est ainsi possible d'injecter une telle solution refroidie sans inconvénient et sans la nécessité d'utiliser une aiguille de forte section. Le refroidissement permet d'obtenir des solutions sensiblement plus fluides. Le retour à une température plus élevée, en particulier proche de la température corporelle, conduit à une augmentation très sensible de la viscosité. De cette façon les solutions injectables selon l'invention une fois injectées retrouvent une viscosité très élevée qui empêche leur diffusion en dehors des sites d'injection et garantissent une rémanence prolongée.

L'invention a donc plus précisément pour objet l'utilisation de compositions pharmaceutiques contenant de l'acide hyaluronique ou l'un de ses sels et du Poloxamer® 407 en vue de la réalisation de solutions injectables très visqueuses destinées à assurer une rémanence prolongée au niveau des articulations et à obtenir en même temps une préparation très élastique, à raison d'un volume de dispersion variant de 1 à 2 mL.

L'invention a également pour objet l'utilisation de compositions cosmétiques contenant de l'acide hyaluronique ou l'un de ses sels et du Poloxamer® 407 en vue de la réalisation de solutions injectables destinées à combler des cicatrices, des rides ou des irrégularités de la peau grâce à une rémanence prolongée dans le derme et à une élasticité particulièrement importante.

Les compositions utilisées assurent ainsi une homéostasie articulaire en rétablissant les propriétés du liquide synovial et de la matrice extra cellulaire des tissus. Les compositions selon l'invention rétablissent à la fois une élasticité proche des valeurs normales, une viscosité comparable à celle du sujet sain, tout en assurant un point de transition faible garantissant l'obtention d'une préparation très élastique.

Les propriétés élastiques des compositions selon l'invention interviennent dans la motricité rapide et les propriétés visqueuses dans la motricité lente.

Les compositions selon l'invention offrent des propriétés rhéologiques proches de celles du liquide synovial du sujet jeune et sain (élasticité à 2,5 Hz chez le sujet sain : 117 ± 13 Pa.s, chez le sujet arthrosique : 8,5± 0,5 Pa.s ; viscosité à 2,5 Hz chez le sujet sain : 45 ± 8,2 Pa.s, chez le sujet arthrosique : 4,8 ± 0,3)

L'acide hyaluronique utilisé dans la présente invention est un polymère présentant une masse molaire plus faible, comprise entre 0,8 et 1,8 millions de daltons. On peut ainsi obtenir des compositions ayant des degrés de viscosité variables en fonction de la variation de la masse molaire. L'acide hyaluronique peut également être utilisé sous forme de sel et notamment d'hyaluronate de sodium, qui présente des propriétés similaires. La concentration d'acide hyaluronique peut varier de 0,5 à 2% en poids des compositions selon l'invention, et de préférence de 0,7 à 0,9%.

Le Poloxamer® 407 est présent dans les compositions selon l'invention dans des proportions qui peuvent varier de 10 à 20% et de préférence de 14 à 18% du poids total de la solution.

Le Poloxamer® 407 intervient pour assurer aux compositions injectables selon l'invention une rémanence sensiblement accrue en ralentissant la dégradation au point d'injection de l'acide hyaluronique, permettant de diminuer le nombre d'injections.

En pratique une composition injectable préférée sera celle contenant 0,8% en poids d'acide hyaluronique et 17,5% en poids de Poloxamer® 407.

Une telle solution présente une viscosité faible à la température de 0 - 10°C. La viscosité augmente ensuite *in situ* au voisinage de la température corporelle.

De manière préférentielle, les compositions selon l'invention sont obtenues en dissolvant dans un volume déterminé de solution tampon saline, de 0,5 à 2 %, d'acide hyaluronique de poids moléculaire de 1,1 millions de Dalton et en dispersant dans cette solution visqueuse de 10 à 20 % en poids de Poloxamer® 407, en maintenant ce mélange à une température voisine de 5°C afin qu'elle présente une viscosité faible. La solution ainsi réalisée est stérilisée par la chaleur humide.

Pour l'utilisation cosmétique et pour la chirurgie plastique, les compositions selon l'invention sont injectées dans les tissus à combler (cicatrices ou rides) à raison d'une ou plusieurs injections par jour jusqu'à ce que la peau, surtout celle du visage ou du cou, retrouve un aspect souple, proche de celui du sujet jeune et sain. De préférence, chaque injection contient de 1 à 5 mL de composition.

L'invention a également pour objet des dispositifs médicaux prêts à l'emploi, contenant des compositions selon l'invention. Un dispositif médical selon l'invention est par exemple constitué d'une seringue pré-remplie, contenant la dose d'injection unitaire d'une composition selon l'invention. De préférence, cette dose varie de 1 à 5 mL.

L'invention définie en termes généraux dans la description qui précède sera mieux comprise à la lumière des exemples figurant ci-après. Ces exemples illustrent l'invention sans toutefois la limiter.

### EXEMPLE : Etude rhéologigue des propriétés des solutions selon l'invention

Les compositions selon l'invention possèdent des propriétés rhéologiques intéressantes, notamment une augmentation de la viscosité en fonction de la température. En effet, l'augmentation de la température amène la formation d'un réseau de gel de plus en plus structuré.

De ce fait, l'élément caractéristique de la présente invention réside dans le fait qu'à la température ambiante (25°C) et même à des températures inférieures, les solutions d'acide hyaluronique peuvent être facilement injectées alors qu'à la température du corps, elles présentent une forme de gel bien structuré, possédant des propriétés viscoélastiques intéressantes.

Les compositions testées sont constituées d'acide hyaluronique à une concentration variant de 0,5 à 2% en poids et de Poloxamer® 407 à une concentration variant de 10 à 20% en poids.

Les différents essais rhéologiques ont été réalisés aux températures de 25 °C et de 37 °C avec des solutions contenant notamment 0,8% d'acide hyaluronique et des concentrations de Poloxamer® 407 s'échelonnant de 10 à 17% (10, 12, 14, 17%).

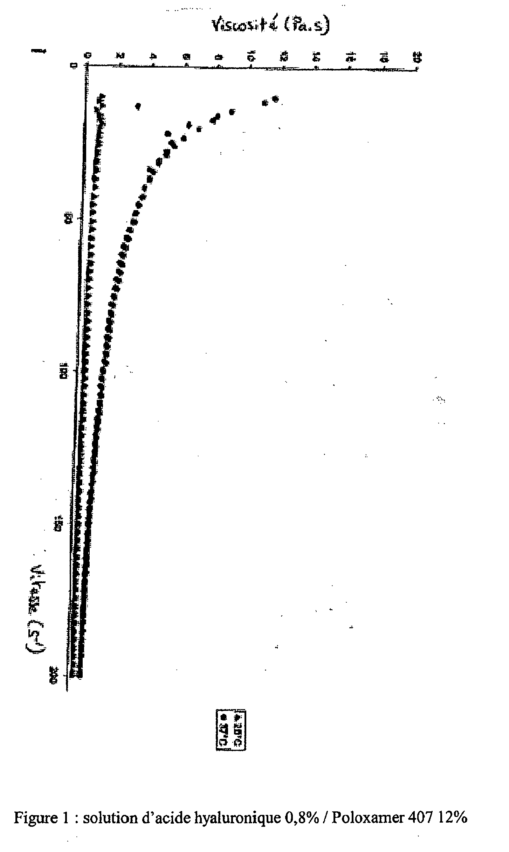

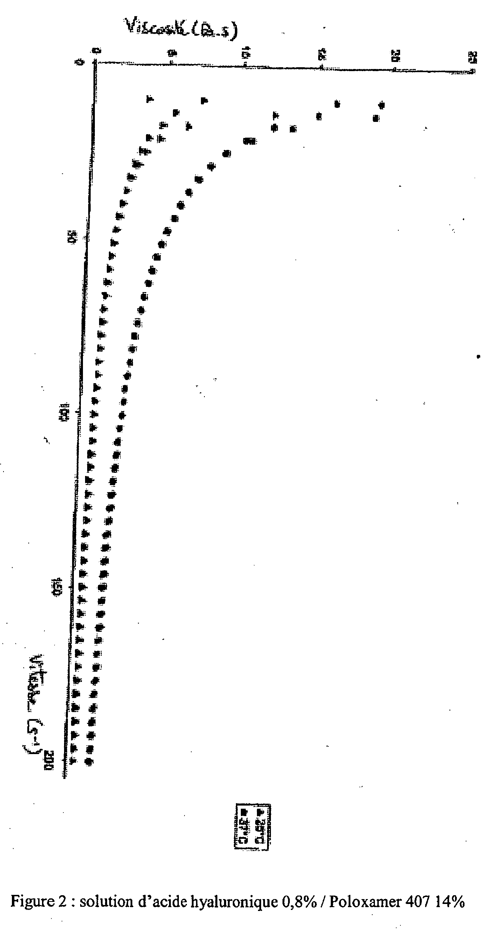

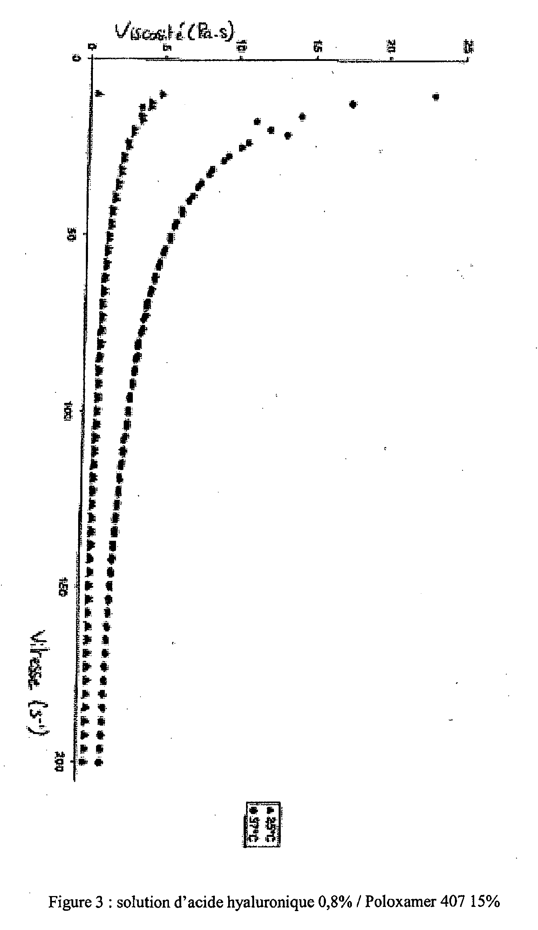

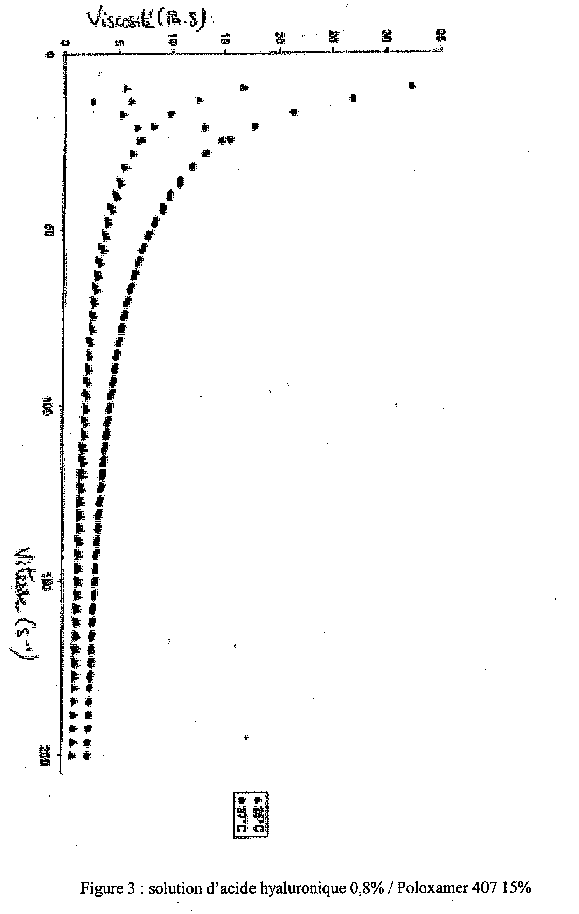

Les rhéogrammes obtenus sont présentés aux figures 1 à 4.

Ils ont été réalisés en balayage vitesse (montée de 0 à 200 s⁻¹ et retour à 0 s⁻¹).

D'autre part, ces compositions présentent une augmentation de viscosité bien marquée à 37 °C. Les viscosités mesurées augmentent avec la température et avec la proportion de Poloxamer® 407 ajouté.

Ces fortes viscosités associées au comportement gel des compositions permettront d'augmenter la rémanence et d'assurer dans le domaine intra-articulaire un effet amortisseur de chocs, très apprécié au moment de la course.

Un résumé des résultats est présenté dans le tableau 1 :

**Tableau 1**

| | **Viscosité (Pa.s) 25 °C, 168 s⁻¹** | **Viscosité (Pa.s) 37°C, 168 s⁻¹** |
|---|---|---|
| AH 0,8% + Pol. 407 12% | 0,41 | 0,92 |
| AH 0,8% + Pol. 407 14% | 0,66 | 1,82 |
| AH 0,8% + Pol. 407 15% | 0,69 | 1,89 |
| AH 0,8% + Pol. 407 17% | 1,40 | 2,77 |

| | | |
|---|---|---|
| AH : acide hyaluronique Pol. 407 : Poloxamer® 407 | | |

## Revendications

1. Compositions pharmaceutiques et/ou cosmétiques et/ou dispositifs médicaux à base d'acide hyaluronique, **caractérisés en ce qu'**ils renferment, au titre de principes actifs, l'acide hyaluronique ou un de ses sels, additionné d'un copolymère en blocs polyoxyéthylène-polyoxypropylène, commercialisé sous la dénomination Poloxamer® 407, dans un solvant aqueux approprié pour une injection intra-articulaire ou subdermique.

2. Compositions et/ou dispositifs selon la revendication 1, dans lesquels la viscosité de la solution diminue avec l'abaissement de la température et augmente avec l'élévation de la température.

3. Compositions et/ou dispositifs selon la revendication 1 et la revendication 2, dans lesquels les solutions d'acide hyaluronique et de Poloxamer® 407 manifestent un maximum de viscosité autour de 37°C et un minimum de viscosité entre 0° et 10°C.

4. Compositions et/ou dispositifs selon l'une des revendications précédentes, dans lesquels la teneur en acide hyaluronique ou en l'un de ses sels se situe entre 0,5 et 2 % en poids de la solution.

5. Compositions et/ou dispositifs selon l'une des revendications précédentes, dans lesquels la teneur en acide hyaluronique ou en l'un de ses sels est comprise entre 0,7 et 0,9 % en poids de la solution.

6. Compositions et/ou dispositifs selon l'une des revendications précédentes, dans lesquels l'acide hyaluronique utilisé est un polymère de poids moléculaire compris entre 0,8 et 1,8 millions de Daltons.

7. Compositions et/ou dispositifs selon l'une des revendications précédentes, dans lesquels le Poloxamer® 407 est présent à une concentration variant de 10 à 20 % en poids.

8. Compositions et/ou dispositifs selon la revendication 7, dans lesquels la concentration en Poloxamer® 407 est comprise de préférence entre 14 et 18 % en poids.

9. Utilisation des compositions selon l'une des revendications 1 à 8, contenant de l'acide hyaluronique ou l'un de ses sels et du Poloxamer® 407, pour réaliser un agent viscoélastique dont la viscosité décroît avec l'abaissement de la température, en vue de l'injection intra-articulaire à raison d'un volume variant de 1 à 5 mL.

10. Utilisation des compositions ou des dispositifs selon l'une des revendications 1 à 8, en chirurgie plastique ou pour des soins cosmétiques.

11. Utilisation des compositions ou des dispositifs selon l'une des revendications 1 à 8, pour des injections sous-cutanées visant à faire disparaître des cicatrices ou des séquelles d'interventions chirurgicales.

12. Utilisation des compositions ou des dispositifs selon l'une des revendications 1 à 8, pour des injections sous-cutanées visant à faire disparaître des rides ou des ridules, ou à retendre la peau.
